(19) Europäisches Patentamt
European Patent Office
Office européen des brevets

(11) Numéro de publication: **0 154 045**
**A2**

(12) # DEMANDE DE BREVET EUROPEEN

(21) Numéro de dépôt: 84201524.0

(22) Date de dépôt: 28.06.82

(51) Int. Cl.[4]: **G 01 N 33/531**

(30) Priorité: 01.07.81 FR 8112934

(43) Date de publication de la demande: 11.09.85 Bulletin 85/37

(84) Etats contractants désignés: AT BE CH DE GB IT LI NL SE

(60) Numéro de publication de la demande initiale en application de l'article 76 CBE: 0 069 018

(71) Demandeur: ROUSSEL-UCLAF
35, boulevard des Invalides
F-75007 Paris(FR)

(72) Inventeur: Jouqey, Alain
137, rue des Pyrénées
F-75020-Paris(FR)

(72) Inventeur: Touyer, Gaetan
2, square Vitrune Tour Giralda
F-75020-Paris(FR)

(74) Mandataire: Niel, Michel et al,
ROUSSEL-UCLAF Boîte postale no 9 111, route de Noisy
F-93230 Romainville(FR)

(54) **Application de dérivés de stilbènes à la préparation d'antigènes et les antigènes obtenus.**

(57) L'invention concerne l'application de dérivés de stilbènes de formule générale IV:

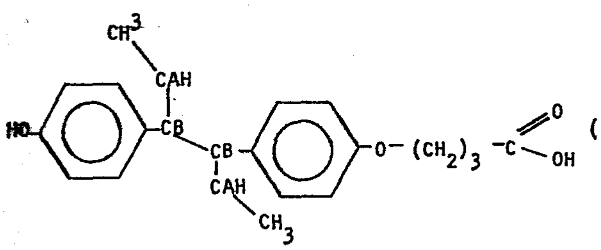
(IV)

dans laquelle A et B représentent chacun un atome d'hydrogène ou représentent ensemble une double liaison, à la préparation d'antigènes, les antigènes obtenus ainsi que l'application desdits antigènes à la préparation d'anticorps.

Application de dérivés de stilbènes à la préparation d'antigènes et les antigènes obtenus.

La présente invention a pour objet l'application de dérivés de stilbènes à la préparation d'antigènes, les antigènes obtenus ainsi que l'application de ces antigènes à la préparation d'anticorps.

L'invention a plus particulièrement pour objet l'application de dérivés de stilbène de formule générale IV :

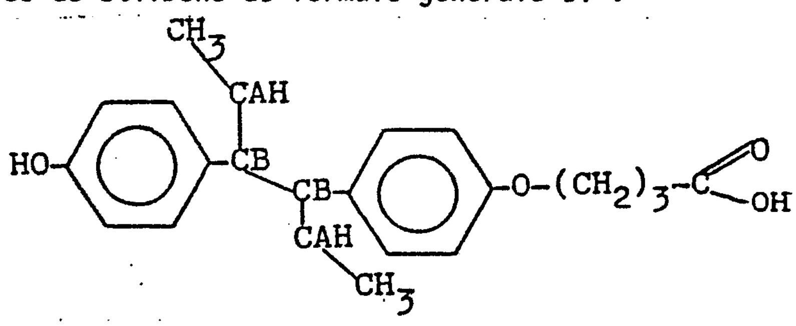

IV.

dans laquelle A et B représentent chacun un atome d'hyrogène ou représentent ensemble une double liaison à la préparation d'antigènes.

Lors de l'application, objet de l'invention, parmi les produits de formule générale IV, on choisit favorablement le diénestrol 4-O-monocarboxypropyl et l'hexestrol 4-O-monocarboxypropyl.

Les produits de formule générale IV sont préparés comme décrit dans la demande de brevet européen 0 069.018.

L'invention a également pour objet, à titre de produits industriels nouveaux, les antigènes obtenus lors de l'application citée ci-dessus.

L'invention a également pour objet l'application des antigènes mentionnés ci-dessus à la préparation des anticorps.

L'application des produits de formule générale IV, objet de la présente invention, est caractérisée en ce que l'on conjugue un de ces produits avec de l'albumine sérique bovine (BSA) ou avec de l'albumine sérique humaine (HSA) et obtient l'antigène cherché.

Dans des conditions préférentielles de mise en oeuvre, l'application des produits de formule générale IV, est caractérisée par les points suivants :

- on fait réagir un produit de formule générale IV :

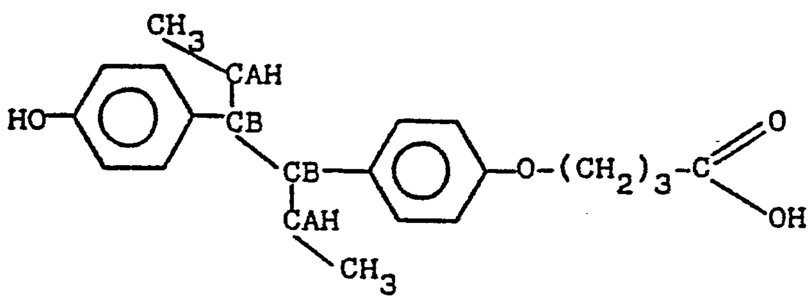

IV

dans laquelle et dans ce qui suit, A et B ont des significations déjà citées, en vue de l'activation de la fonction carbonyle, avec un halogénoformiate d'alcoyle, en présence d'une base tertiaire, en milieu anhydre et sous atmosphère inerte, et obtient l'anhydride mixte de formule V :

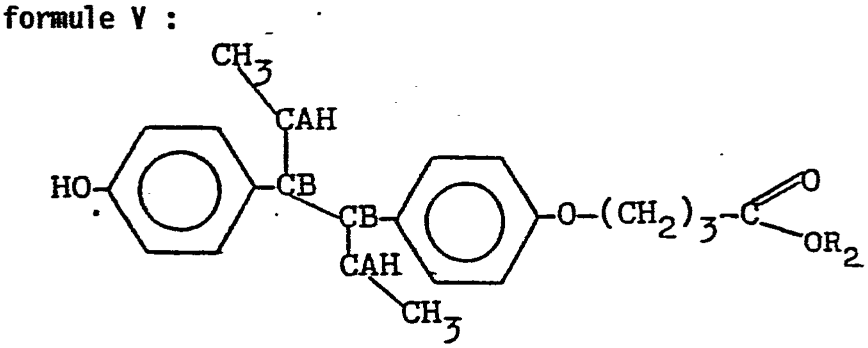

V

dans laquelle $R_2$ est un groupement activateur de la fonction carbonyle de formule -CO-O-ALK, ALK étant un radical alcoyle ayant au plus 6 atomes de carbone,

- que l'on conjugue avec l'albumine sérique bovine (BSA) ou humaine (HSA) et obtient l'antigène cherché.

L'application des produits de formule générale IV est encore caractérisée par les modes d'exécution suivants :

- l'halogéno formiate d'alcoyle que l'on fait réagir avec le produit de formule générale IV est le chloroformiate d'isobutyle et on opère en présence de tri-n-butylamine, dans le dioxane anhydre et sous atomosphère inerte vers + 4° C ;

- on fait réagir l'anhydride mixte de formule générale V avec l'albumine sérique bovine ou humaine en ayant préalablement dissous cette dernière dans un mélange eau-dioxane sous atmosphère inerte.

L'application décrite permet d'obtenir respectivement

- au départ du diénestrol 4-0-monocarboxypropyl de formule :

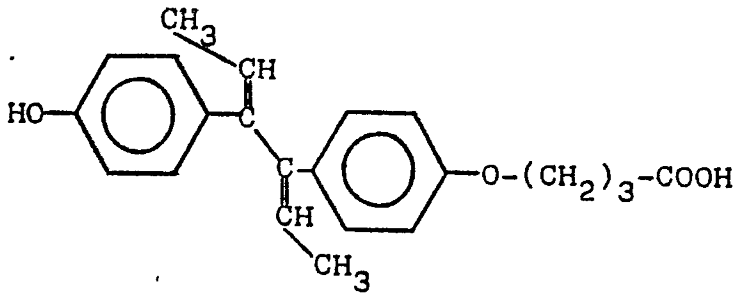

- les antigènes de formules :

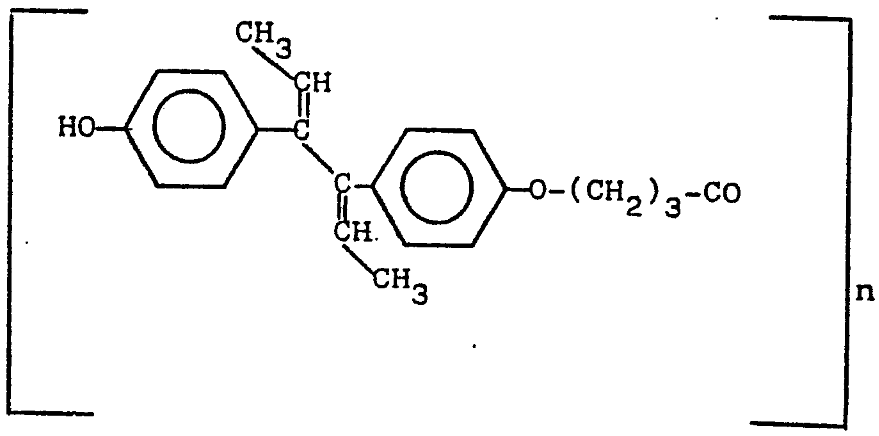

VII

-(BSA)

dans laquelle n = 20 à 40 ; et

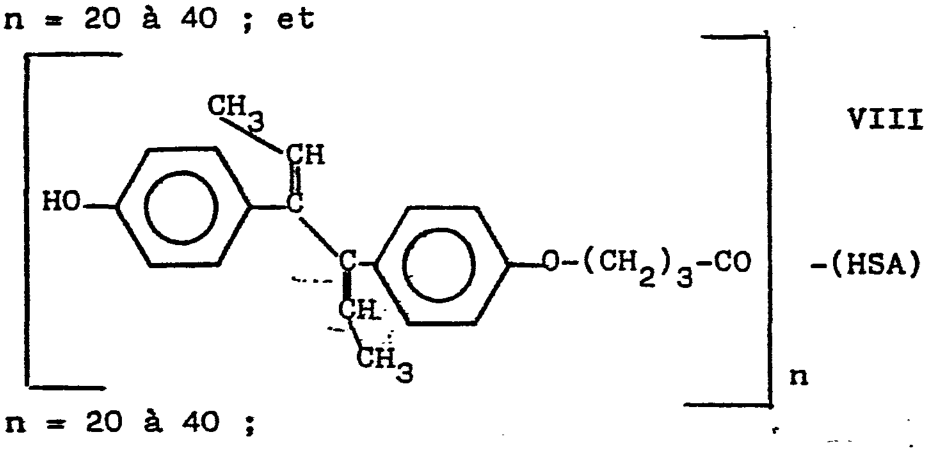

VIII

dans laquelle n = 20 à 40 ;

- et au départ de l'hexestrol 4-O-monocarboxypropyl de formule :

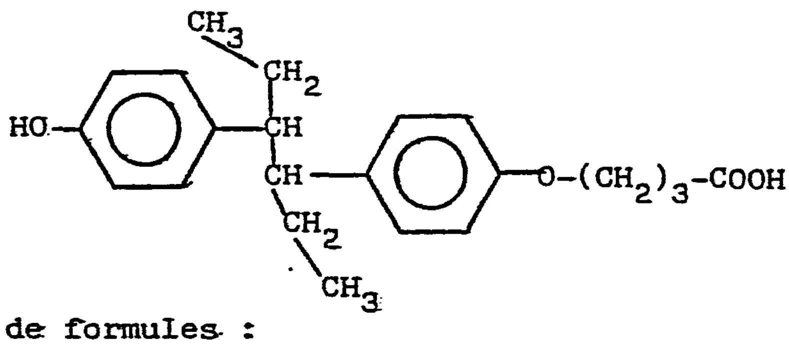

les antigènes de formules :

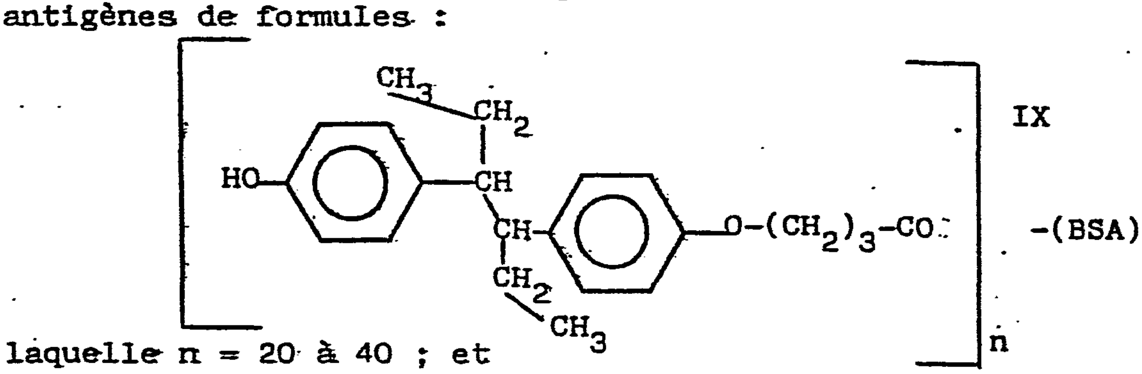

IX

dans laquelle n = 20 à 40 ; et

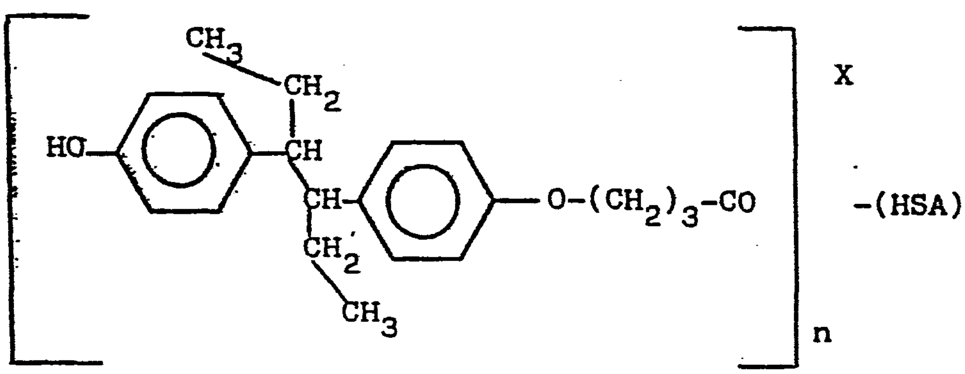

X

dans laquelle n = 20 à 40.

L'invention a également pour objet, à titre de produits industriels nouveaux, les antigènes obtenus lors de l'application décrite ci-dessus, c'est-à-dire les produits de formules générales VII, VIII, IX et X précités.

L'invention a également pour objet l'appliation des antigènes ci-dessus, à la préparation des anticorps.

Les antigènes de formules VII, VIII, IX et X précités, également objet de l'invention, sont utilisés pour le développement d'anticorps. Injectés chez l'animal en présence d'un adjuvant, ils permettent, en effet, d'obtenir chez celui-ci des sérums contenant des anticorps contre l'hexestrol, le diénestrol, ainsi que contre le diéthylstilbestrol.

Les exemples suivants illustrent l'invention sans toutefois la limiter.

Exemple I : Préparation d'antigène par conjugaison du diénestrol 4-O-monocarboxypropyl et d'albumine sérique bovine (BSA).

Stade A : Anhydride mixte du diénestrol 4-O-monocarboxypropyl avec le formiate d'isobutyle.

On dissout 352 mg de diénestrol 4-O-monocarboxypropyl dans 10 ml de dioxane, puis ajoute 0,46 ml de tri-n-butylamine et agite le mélange sous atmosphère inerte. On abaisse la température à 12° C et ajoute 0,126 ml de chloroformiate d'isobutyle et maintient l'agitation pendant 30 minutes à 12° C. On obtient la solution du produit cherché que l'on utilise telle quelle pour le stade suivant.

Le produit de départ a été préparé comme décrit dans la de brevet européen 0 069 018.

Stade B : Dissolution de la BSA.

On prépare un mélange de 44 ml d'eau glacée et de 4 ml de dioxane, puis ajoute, sous agitation et à une température voisine de 0° C, 1,54 g d'albumine sérique bovine. On transvase ensuite la solution résultante dans un dispositif à dialyse et laisse pendant 72 heures en dialyse à une température située entre 1° et 5° C, avec un contre-courant de 30 l d'eau.

On extrait ensuite le dialysat obtenu (pH 7,9) par le chloroforme glacé, sépare les phases organiques, les lave à l'eau. On réunit les phases aqueuses, les congèle, puis les lyophilise pendant 20 heures sous vide de 0,01 mm Hg, et obtient 1,4 g de produit.

Stade C : Conjugaison :

On dissout à 0° C le résidu obtenu ci-dessus dans 40 ml de dioxane glacé, puis ajoute 1,35 ml de NaOH N et aussitôt l'anhydride

mixte préparé au stade A. On ajuste le pH à 8,9 par addition d'acide chlorhydrique N/10 et abandonne le mélange réactionnel pendant 4 heures sous agitation, à une température proche de 0° C. On le soumet ensuite à la dialyse pendant 18 heures, à une température comprise entre 1° C et 5° C, à contre-courant d'eau. Après le passage de 30 litres d'eau, le dialysat est transvasé, acidifié à pH 4,1 par l'acide chlorhydrique N, puis N/10, congelé pendant une nuit vers -18° C, puis dégelé lentement sans dépasser + 5° C.

Un précipité se forme et la solution surnageante est éliminée par filtration. Le précipité est repris par 100 ml d'une solution de bicarbonate de sodium à 1 % et la solution est soumise à la dialyse pendant 72 heures dans les conditions précitées. On extrait ensuite le dialysat par le chloroforme glacé, et lave la phase organique à l'eau. Les phases aqueuses réunies sont congelées, puis lyophilisées pendant 20 heures sous un vide de 0,01 mm Hg pour donner 1,4 g d'antigène cherché.

Ce produit contient 37 à 38 groupes diénestrol liés par mole.

Exemple II : Préparation d'antigène par conjugaison d'hexestrol -4-O-monocarboxypropyl et d'albumine sérique bovine (BSA).

En procédant comme décrit à l'exemple I, en partant de 356 mg d'hexestrol-4-O-monocarboxypropyl et de 1,54 g d'albumine sérique bovine (BSA), on obtient 1,3 g d'antigène cherché. Ce produit contient 31 à 32 groupes d'hexestrol par mole.

Exemple III : Préparation d'antigène par conjugaison d'hexestrol -4-O-monocarboxypropyl et d'albumine sérique humaine (HSA).

En procédant comme décrit à l'exemple I, en partant de 356 mg d'hexestrol-4-O-monocarboxypropyl et 1,54 g d'albumine sérique humaine (HSA) on obtient 1,17 g de l'antigène cherché. Ce produit contient 21 groupes d'hexestrol liés par mole.

Le produit de départ des exemples II et III a été préparé comme décrit dans la demande de brevet européen 0 069 018.

0154045

**Revendications** :

1) Application des produits de formule générale IV :

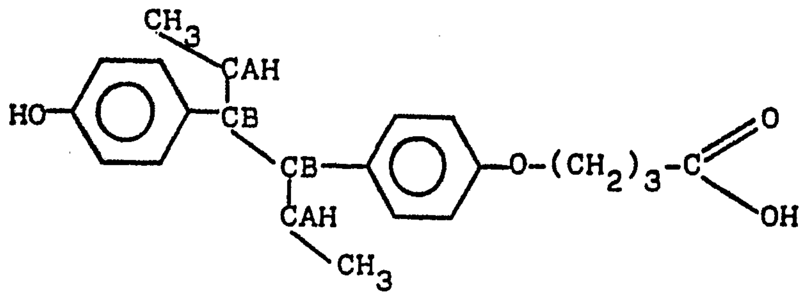

IV

dans laquelle A et B représentent chacun un atome d'hydrogène ou représentent ensemble une double liaison à la préparation d'antigènes, caractérisée en ce que l'on conjugue un de ces produits avec de l'albumine sérique bovine (BSA) ou avec de l'albumine sérique humaine (HSA) et obtient l'antigène cherché.

2) Application selon la revendication 1, caractérisée en ce que :

- on fait réagir un produit de formule générale IV :

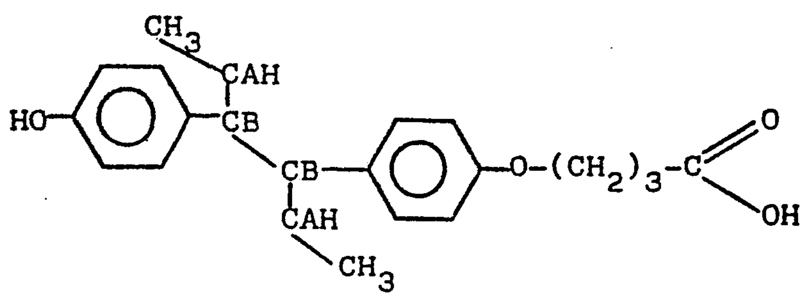

IV

dans laquelle et dans ce qui suit, A et B ont des significations déjà citées, en vue de l'activation de la fonction carbonyle, avec un halogénoformiate d'alcoyle, en présence d'une base tertiaire, en milieu anhydre et sous atmosphère inerte et obtient l'anhydride mixte de formule V :

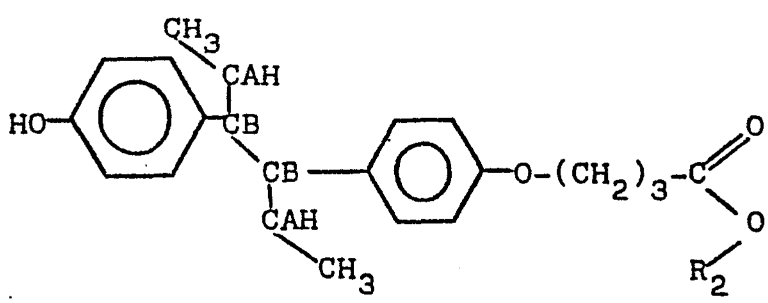

V

dans laquelle $R_2$ représente un radical de formule -CO-O-Alk, Alk étant un radical alcoyle ayant au plus 6 atomes de carbone,

- que l'on conjugue avec l'albumine sérique bovine (BSA) ou humaine

(HSA) et obtient l'antigène cherché.

3) Application selon la revendication 2, caractérisée en ce que :

- l'halogéno formiate d'alcoyle que l'on fait réagir avec le produit de formule générale IV est le chloroformiate d'isobutyle et que l'on opère en présence de tri-n-butylamine, dans le dioxane anhydre et sous atmosphère inerte vers + 4° C ;

- on fait réagir l'anhydride mixte de formule générale V, avec l'albumine sérique bovine ou humaine en ayant préalablement dissous cette dernière dans un mélange eau-dioxane sous atomosphère inerte.

4) A titre de produits nouveaux,

- les antigènes de formules :

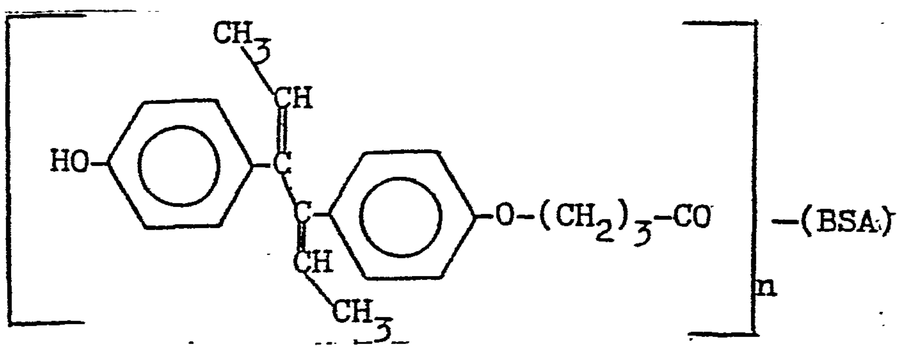

VII

dans laquelle n = 20 à 40 ;

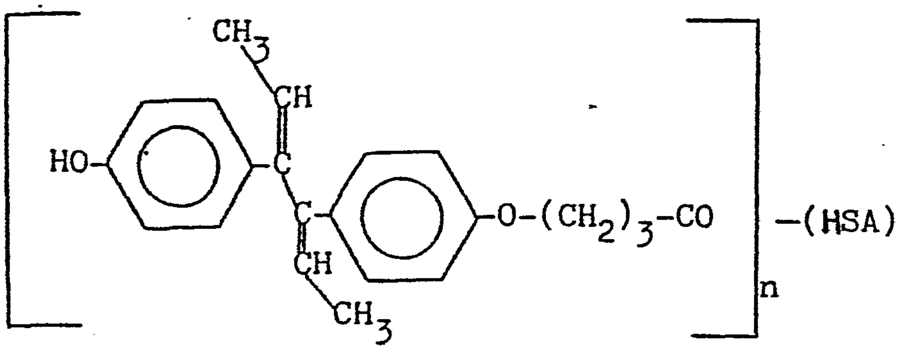

VIII

dans laquelle n = 20 à 40 ;

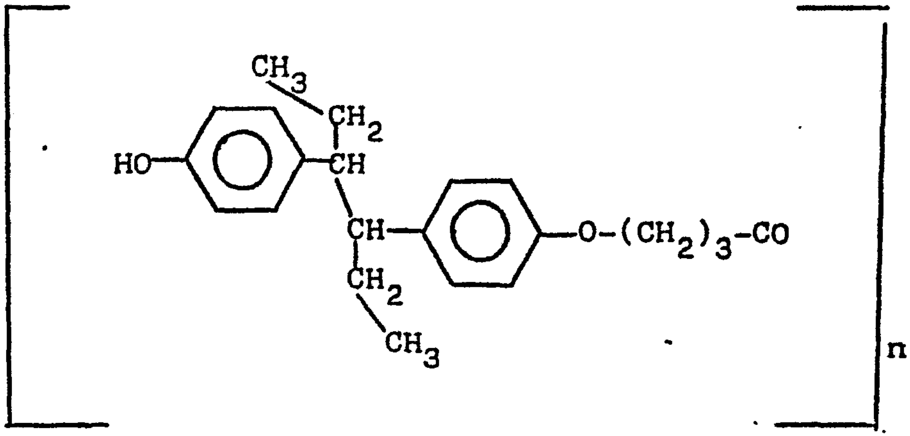

n

IX

-(BSA)

dans laquelle n = 20 à 40 et

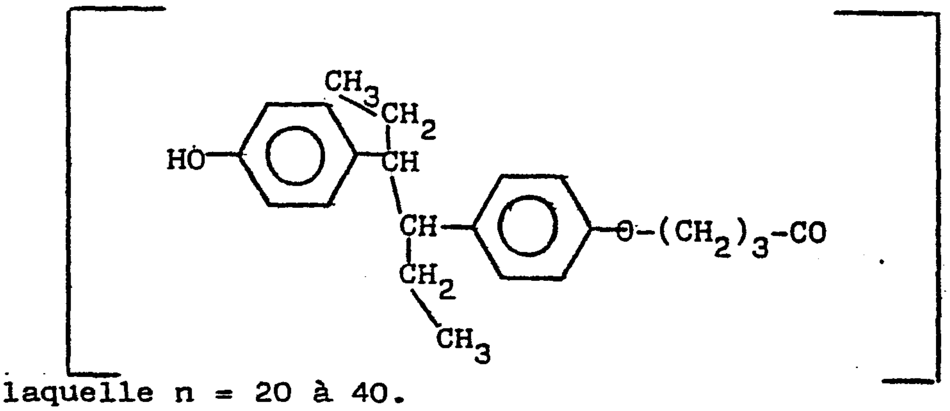

n

X

-(HSA)

dans laquelle n = 20 à 40.

5) Application des antigènes selon la revendication 4 à la préparation des anticorps.

1) Application des produits de formule générale (IV)

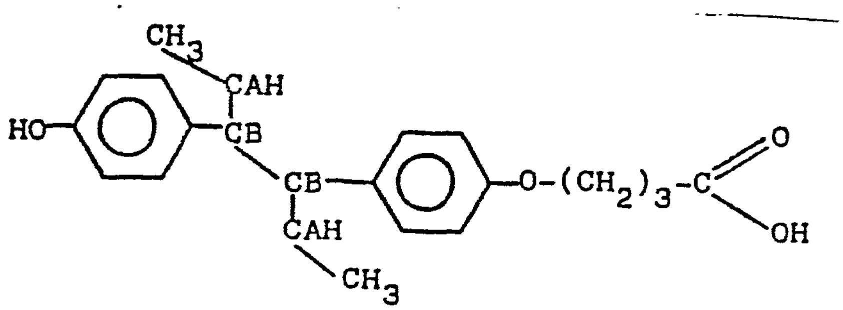

IV

dans laquelle A et B représentent chacun un atome d'hydrogène ou représentent ensemble une double liaison à la préparation d'antigènes, caractérisée en ce que l'on conjugue un de ces produits avec de l'albumine sérique bovine (BSA) ou avec de l'albumine sérique humaine (HSA) et obtient l'antigène cherché.

2) Application selon la revendication 1, caractérisée en ce que :

- on fait réagir un produit de formule générale IV :

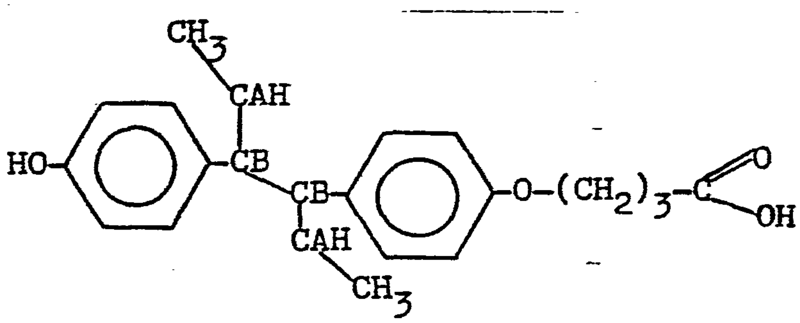

IV

dans laquelle et dans ce qui suit, A et B ont des significations déjà citées, en vue de l'activation de la fonction carbonyle, avec un halogénoformiate d'alcoyle, en présence d'une base tertiaire, en milieu anhydre et sous atmosphère inerte et obtient l'anhydride mixte de formule V :

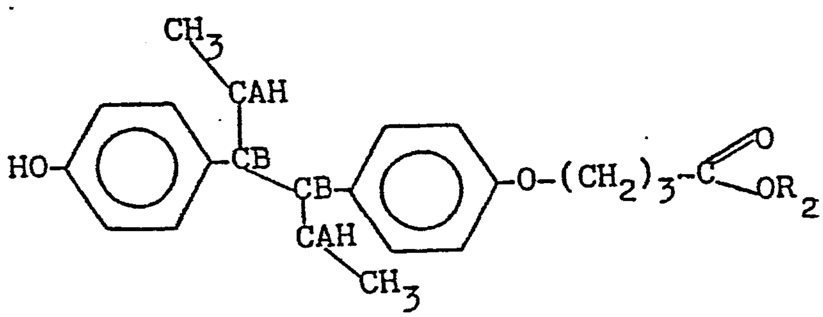

V

dans laquelle $R_2$ représente un radical de formule -CO-O-Alk, Alk étant un radical alcoyle ayant au plus 6 atomes de carbone,
- que l'on conjugue avec l'albumine sérique bovine (BSA) ou humaine (HSA) et obtient l'antigène cherché.

3) Application selon la revendication 2, caractérisée en que :
- l'halogéno formiate d'alcoyle que l'on fait réagir avec le produit de formule générale IV est le chloroformiate d'isobutyle et que l'on opère en présence de tri-n-butylamine dans le dioxane anhydre et sous atomosphère inerte vers + 4° C ;
- on fait réagir l'anhydride mixte de formule générale V, avec l'albumine sérique bovine ou humaine en ayant préalablement dissous cette dernière dans un mélange eau-dioxane sous atomosphère inerte.

4) Application des antigènes tels qu'obtenus à la revendication 1, 2 ou 3 à la préparation des anticorps.